# EUROPÄISCHE PATENTSCHRIFT

(11) **EP 0 980 242 B1**
(45) Veröffentlichungstag und Bekanntmachung des Hinweises auf die Patenterteilung: **05.02.2003**
(21) Anmeldenummer: 98919202.6
(22) Anmeldetag: 01.04.1998
(51) Int. Cl.: A61K 31/00, A61K 31/194, A61P 37/06

(54) **VERWENDUNG VON FUMARSÄUREDERIVATEN ZUR BEHANDLUNG VON AUTOIMMUNERKRANKUNGEN**
USE OF FUMARIC ACID DERIVATIVES FOR THE TREATMENT OF AUTOIMMUNE DISEASES
UTILISATION DE DERIVES D'ACIDE FUMARIQUE POUR LE TRAITEMENT DE MALADIES AUTO-IMMUNES

(30) Priorität: 20.05.1997 DE 19721099
(43) Veröffentlichungstag der Anmeldung: 23.02.2000
(73) Patentinhaber: Fumapharm AG, 5630 Muri (CH)
(72) Erfinder: JOSHI, Rajendra, K., CH-8048 Zürich (CH); STREBEL, Hans-Peter, CH-5630 Muri (CH)
(74) Vertreter: Schwabe - Sandmair - Marx
(86) Internationale Anmeldenummer: EP9801894
(87) Internationale Veröffentlichungsnummer: WO98052549

(56) Entgegenhaltungen:
- EP-A- 0 312 697
- EP-A- 0 518 388
- EP-A- 0 793 966
- WO-A-96/01122
- WO-A-96/08970
- CN-A- 1 125 141
- DE-A- 2 530 372
- DE-A- 2 621 214
- GB-A- 2 291 422
- M. BACHARACH-BUHLES ET AL.: "The Effect of Fumaric Acid Esters and Dithranol on Acanthosis and Hyperproliferation in Psoriasis Vulgaris" ACTA DERM. VENEREOL., Bd. 76, Nr. 03, Mai 1996, Seiten 190-193, XP002088939
- M. BACHARACH-BUHLES ET AL.: "Fumaric Acid Esters (FAEs) Suppress CD 15- and ODP 4-positive Cells in Psoriasis" ACTA DERM. VENEREOL. SUPPL., Bd. 186, 1994, Seiten 79-82, XP002088940
- T. HUNZIKER ET AL.: "Psoriasis, eine Autoimmunkrankheit?" THER.UMSCH., Bd. 50, Nr. 2, 1993, Seiten 110-113, XP002088941
- H.M.OCKENFELS ET AL.: "The antipsoriatic agent dimethylfumarate immunomodulates T-cell cytokine secretion and inhibits cytokines of the psoriatic cytokine network" BRITISH JOURNAL OF DERMATOLOGY, Bd. 139, Nr. 3, September 1998, Seiten 390-395, XP002089713 & CONGRESS: "PSORIASIS: FROM GENE TO CLINIC", 5. - 7. Dezember 1996,
- W. BAYARD ET AL.: "Perorale Langzeitbehandlung der Psoriasis mit Fumarsäurederivaten" HAUTARZT, Bd. 38, Nr. 5, 1987, Seiten 279-285, XP002076555
- H.J.PROCHASKA ET AL.: "Elevation of Glutathion Levels by Phase II Enzyme Inducers: Lack of Inhibition of Human Immunodeficiency Virus Type 1 Replication in Chronically Infected Monocytoid Cells" MOL.PHARMACOL., Bd. 45, Nr. 5, Mai 1994, Seiten 916-921, XP002088942
- B.SEBÖK ET AL.: "Antiproliferative and cytotoxic profiles of antipsoriatic fumaric acid derivatives in keratinocyte cultures" EUR.J.PHARMACOL., Bd. 270, Nr. 1, 3. Januar 1994, Seiten 79-87, XP002088943
- H.B. THIO ET AL.: "Fumaric acid derivatives evoke a transient increase in intracellular free calcium concentration and inhibit the proliferation of human keratinocytes" BR.J.DERMATOL., Bd. 131, Nr. 6, Dezember 1994, Seiten 856-861, XP002088944
- R. KIEHL ET AL.: "A Defective Purine Nucleotide Synthesis Pathway in Psoriatic Patients" ACTA DERM. VENEREOL., Bd. 72, Nr. 4, August 1992, Seiten 253-255, XP002088945
- B. SEBÖK ET AL.: "Effect of Fumaric Acid, Its Dimethylester, and Topical Antipsoriatic Drugs on Epidermal Differentiation in the Mouse Tail Model" SKIN PHARMACOL., Bd. 9, Nr. 2, März 1996 - April 1996, Seiten 99-103, XP002088946
- A. LAHTI ET AL.: "Contact urticaria from diethyl fumarate" CONTACT DERMATITIS, Bd. 12, Nr. 3, März 1985, Seiten 139-140, XP002088947
- L FLIEGNER ET AL.: "Osteomalazie als offenbar seltene Nebenwirkung der oralen Fumarsäuretherapie" HAUTARZT, Bd. 43, Nr. 9, September 1992, Seiten 554-560, XP002088948
- A. SADJAK ET AL.: "Nephrotoxische Wirkung von Fumarsäurederivaten" DTSCH. MED. WOCHENSCHR., Bd. 116, Nr. 12, 22. März 1991, Seite 478 XP002088949
- A. LAHTI ET AL.: "Acetylsalicylic acid inhibits non-immunologic contact urticaria" CONTACT DERMATITIS, Bd. 16, Nr. 3, März 1987, Seiten 133-135, XP002088950
- P. DÜCKER ET AL.: "Zwei Fälle von Nebenwirkungen einer Fumarsäureester - Lokaltherapie" Z. HAUTKR., Bd. 65, Nr. 8, August 1990, Seiten 734-736, XP002088951
- D.N.KOLBACH ET AL.: "Fumaric acid therapy in psoriasis: Results and side effects of 2 years of treatment" J. AM. ACAD. DERMATOL., Bd. 27, Nr. 5, Part I, November 1992, Seiten 769-771, XP002089560
- M. VANDERMEEREN ET AL.: "Dimethylfumarate Is an Inhibitor of Cytokine-induced E-Selectin, VCAM-1, and ICAM-1 Expression in Human Endothelial Cells" BIOCHEM.BIOPHYS.RES.COMMUN., Bd. 234, Nr. 1, 8. Mai 1997, Seiten 19-23, XP002089561
- C. NIEBOER ET AL.: "Systemic therapy with fumaric acid derivatives: New possibilities in the treatment of psoriasis" J. AM. ACAD. DERMATOL., Bd. 20, Nr. 4, April 1989, Seiten 601-608, XP002089562

## Beschreibung

Die Erfindung betrifft die Verwendung bestimmter Fumarsäuremonoalkylester als Salze allein oder in Kombination mit einem Dialkylfumarat zur Herstellung von pharmazeutischen Zubereitungen zur Behandlung bestimmter Autoimmune Krankheiten wie Polyarthirits, Multiplen Sklerose oder sowie von Graft-versus-Host-Reaktionen. Die Erfindung betrifft ferner die Verwendung einer oder mehrerer Fumarsäuremonoalkylester Form von freien Säuren gegebenenfalls zusammen mit Dialkylfumarat zur Herstellung eines Arzneimittels zur Behandlung der Polyarthritis, Multiplen Sklerose, Graft-versus-Host-Reaktionen und anderen Autoimmunkrankheiten. Diese Zubereitungen enthalten keine Fumarsäure per se. Die erfindungsgemäße Verwendung erstreckt sich auch auf die Behandlung von juvenilem Diabetes, der Hashimoto-Thyreoiditis, der Grave's disease (Graves Krankheit oder Basedow Krankheit), des systemischen Lupus erythematodes (SLE), des Sjögren Syndroms (Sjogren's Syndrome), der pemiziösen Anämie und der chronischen aktiven (=lupoiden) Hepatitis. Die erfindungsgemäßen Gegenstände sind in den Ansprüchen im einzelnen gekennzeichnet.

Pharmazeutische Zubereitungen, die nach Verabreichung bei ihrem biologischen Abbau in den Zitronensäurezyklus einmünden oder diesem angehören, gewinnen, zumeist in hoher Dosierung, immer mehr an therapeutischem Wert, da man mit ihrer Hilfe kryptogenetisch bedingte Krankheiten zu lindern oder zu heilen vermag.

So hemmt Fumarsäure das Wachsturn des Ehrlich-Ascites-Tumors bei Mäusen, vermindert die toxischen Effekte von Mitomycin C und Aflatoxin (K. Kuroda, M. Akao, Biochem. Pharmacol. 29, 2839-2844 (1980) / Gann. 72, 777-782 (1981) / Cancer Res. 36, 1900-1903 (1976)) und besitzt eine antipsoriatische sowie antimikrobielle Wirkung [C. N. Huhtsnen, J. Food Sci. 48, 1574 (1983) / M. N. Islam, U.S.-Patent 4 346 118 vom 24. August 1982 / C.A. 97, 161317b (1982)].

Hohe Verabreichungsdosen von Fumarsäure oder ihren bisher bekannten Derivaten wie Dihydroxyfumarsäure, Fumaramid und Fumaronitril besitzen bei parenteraler, dermaler, insbesondere aber peroraler Verabreichung eine derart unzumutbare Nebenwirkungsrate und hohe Toxizität (P. Holland, R.G. White, Brit. Dermatol. 85, 259-263 (1971) / M. Hagedorn, K.W. Kalkoff, G. Kiefer, D. Baron, J. Hug, J. Petres, Arch. Derm. Res. 254, 67-73 (1975)), daß bisher meist von einer solchen Therapie abgesehen werden musste.

In der europäischen Patentanmeldung 18 87 49 sind bereits Fumarsäurederivate und sie enthaltende pharmazeutische Zubereitungen zur Behandlung der Psoriasis beschrieben. Aus der DE-A-25 30 372 sind pharmazeutische Zubereitungen zur Behandlung der Psoriasis bekannt, die eine Mischung aus Fumarsäure und weiteren Fumarsäurederivaten enthaften. Der Anteil an freier Fumarsäure ist bei diesen Arzneimitteln obligatorisch.

Die DE-A-26 21 214 beschreibt Arzneimittel zur Behandlung der Psoriasis, die den Fumarsäurermonoethylester und dessen Mineralsalze als Wirkstoff enthalten. Aus der Publikation "Hautarzt (1987) 279-285" ist die Verwendung von Fumarsäuremonoethylestersalzen (Ca, Zn, Mg) und des Fumarsäuredimethylesters für die Psoriasisbehandlung bekannt. Aus dem Patent EP0312 697 B1 sind pharmazeutische Zubereitungen zur Behandlung der Psoriasis, psoriatischen Arthritis, Neurodermitis und Enteritis regionalis Crohn bekannt, die eine Mischung aus Fumarsäuremonoalkylestersalzen und einem Fumarsäurediester enthalten.

Weiterhin beschreiben die Artikel von Bacharach-Buhles M. et al. in "ACTA DERM VENEREOL", Band 76, 1996, Seiten 190-193, und "ACTA DERM VENEREOL", Ergänzungsband 186, 1994, Seiten 79-82, die Auswirkung der Behandlung von Psoriasis-Plaques mit Fumarsäureestern, wobei ein immun-supressiver oder immun-modulativer Mechanismus vermutet wird. Ein spezieller Wirkmechanismus ist nicht angegeben.

Th. Hunziker et al. beschreiben jedoch in ihrem Artikel "Psoriasis, eine Autoimmunkrankheit?" in THERAPEUTISCHE UMSCHAU, Band 50, 1993, Seiten 110-113, dass die Psoriasis nach derzeitigem Kenntnisstand nicht zu den Autoimmunerkrankungen gerechnet werden kann.

Es wurde nunmehr überraschend bei in-vitro-Untersuchungen sowie im Tierversuch herausgefunden, daß eine Behandlung einer Autoimmunerkrankung, ausgewählt aus der Gruppe, bestehend aus der Polyarthritis, Multiplen Sklerose, Graft-versus-Host-Reaktionen, des juvenilen Diabetes, der Hashimoto-Tyreoiditis, der Grave's disease (Graves Krankheit oder Basedow Krankheit), des systemischen Lupus erythematodes (SLE), dem Sjögren Syndrom (Sjogren's Syndrome), der perniziösen Anämie und der chronischen aktiven (= lupoiden) Hepatitis, durch pharmazeutische Zubereitungen möglich ist, die die Verwendung einer oder mehrerer Verbindungen aus der Gruppe der Natrium-, Kalium-, Calcium-, Magnesium-, Zink- und Eisensalze von Fumarsäuremonoalkylester der allgemeinen Formel 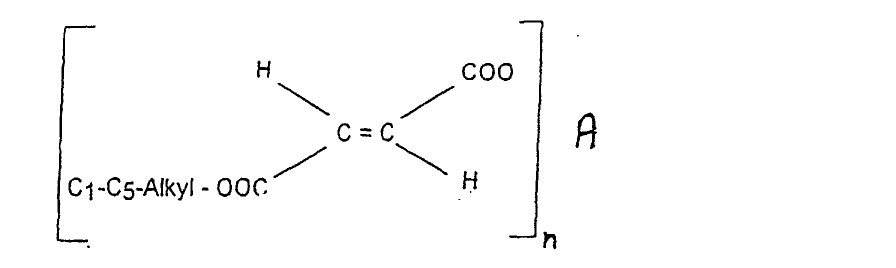 gegebenenfalls im Gemisch mit Dialkylfumarat der Formel. 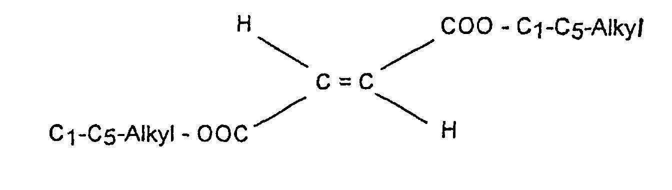 ggf. neben üblichen pharmazeutischen Hilfs- und Trägerstoffen vorsehen,
wobei A ein zweiwertiges Kation der Reihe Ca, Mg, Zn oder Fe bzw. ein einwertiges Kation aus der Reihe Kalium oder Natrium und n 1 oder 2 je nach Art des Kations bedeuten.

Es wurde ebenfalls eine Wirkung bei der Behandlung der oben genannten Autoimmunerkrankungen durch pharmazeutische Zubereitungen gefunden, die die Verwendung einer oder mehrerer Verbindungen aus Alkylhydrogenfumarsäure der allgemeinen Formel 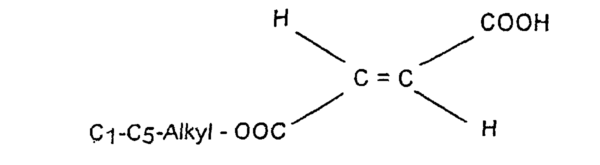 gegebenenfalls im Gemisch mit Dialkylfumarat der Formel 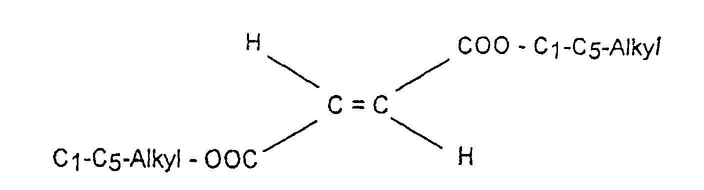 neben ggf. üblichen pharmazeutischen Hilfs- und Trägerstoffen vorsehen.

Bevorzugte Zubereitungen enthalten das Calciumsalz des Fumarsäure-Monomethylesters, das Calciumsalz des Fumarsäure-Monomethylesters im Gemisch mit Dimethylfumarat bzw. den jeweiligen Salzen des Fumarsäuremonoethylesters.

Zur Verabreichung sind besonders Zubereitungen geeignet, die das Calciumsalz des Fumarsäure-Monoalkylesters bzw. den Fumarsäurealkylester in Form der freien Säure in einer Menge von 10 bis 300 mg enthalten, wobei das Gesamtgewicht der Wirkstoffe 10 bis 300 mg beträgt.

Weitere bevorzugte orale Verabreichungsformen enthalten 10 bis 290 Gew.-Teile des Calciumsalzes des Fumarsäure-Monoalkylesters und 290 bis 10 Gew.-Teile Dimethylfumarat sowie 1 bis 50 Gew.-Teile des Zinksalzes des Fumarsäure-Monoalkylesters oder 1 bis 250 Gew.-Teile des Calciumsalzes des Fumarsäure-Monoalkylesters, 250 bis 10 Gew.-Teile Dimethylfumarat, 1 bis 50 Gew.-Teile des Magnesiumsalzes des Fumarsäure-Monoalkylesters und 1 bis 50 Gew.-Teile des Zinksalzes des Fumarsäure-Monoalkylesters bzw. des Monomethylesters, wobei jeweils das Gesamtgewicht der Wirkstoffe 30 bis 300 mg beträgt.

Bevorzugte Zubereitungen enthalten auch das Methylhydrogenfumarat in einer Menge von 10 bis 300 mg.

Ganz besonders bevorzugt liegen Zubereitungen in Form von Mikrotabletten oder Mikropellets vor.

Für den systemischen Einstieg in die Behandlung wie auch umgekehrt für den Ausstieg (ausschleichende Dosierungen) ist eine niedrige Dosierung vorteilhaft, die beispielsweise aus 30.0 mg Dimethylfumarat, 20.0 mg des Calciumsalzes von Monoethylfumarat und 3.0 mg des Zinksalzes von Monoethylfumarat bzw. Monomethylfumarat enthält.

Für die therapeutische Dosierung nach einer Einstiegsphase kann beispielsweise eine Dosierung von 120.0 mg Dimethylfumarat, 87.0 mg des Calciumsalzes des Monoethylfumarats und 3.0 mg des Zinksalzes des Monoethylfumarats bzw. des Monomethylfumarats zur Anwendung kommen.

Die in den Zubereitungen enthaltenen Fumarsäurederivate werden beispielsweise dadurch erhalten, daß man eine Verbindung der folgenden Formel 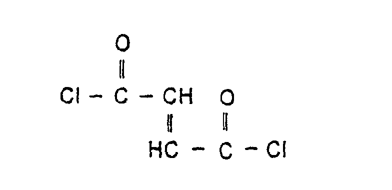
a) mit 2 Mol Alkylalkohol (ROH) in bekannter Weise zum Diester kondensiert und anschließend kontrolliert zum Monoester hydrolysiert, oder
b) mit 1 Mol eines entsprechenden Alkylalkohols (ROH) in üblicher Weise kondensiert und das erhaltene Monosäurechlorid zur Säure hydrolysiert, oder
c) die Fumarsäure direkt mit 2 Mol Alkylalkohol (ROH) in bekannter Weise zu dem jeweiligen Diester kondensiert und anschließend kontrolliert zum Monoester hydrolysiert, oder
d) Maleinsäure oder Maleinsäureanhydrid direkt mit 1 - 2 Mol des entsprechenden Alkylalkohols (ROH) in bekannter Weise zu einem Mono- oder Diester kondensiert und anschließend katalytisch zum entsprechenden Fumarsäurederivat isomerisiert.

Die Salze der Fumarsäuremonoalkylester können dadurch erhalten werden, daß man eine Verbindung der allgemeinen Formel 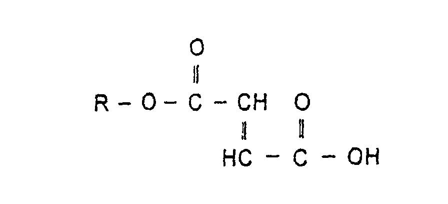 in der R eine C₁-C₅-Alkylgruppe bedeutet, mit äquivalenten Molmengen Na-, K-, Fe-, Ca-, Mg- oder Zn-Hydroxid oder -Oxid in Toluol zur Umsetzung bringt und das während der Reaktion gebildete Wasser entfernt.

Bei besonders bevorzugten Verwendungen werden insbesondere Arzneimittel mit den folgenden Wirkstoffen in den bezeichneten Dosierungen und Mengenverhältnissen eingesetzt:
als pharmazeutische Zubereitungsform zur oralen Verabreichung in Form von Tabletten oder Kapseln nach Anspruch 1, die dadurch gekennzeichnet sind, daß sie das Calciumsalz des Fumarsäuremonomethylesters in einer Menge von 10 bis 300 mg enthält, wobei das Gesamtgewicht der Wirkstoffe 10 bis 300 mg beträgt oder als
pharmazeutische Zubereitungsform zur oralen Verabreichung in Form von Tabletten oder Kapseln, die dadurch gekennzeichnet sind, daß sie 10 bis 290 Gewichtsteile des Calciumsalzes des Fumarsäuremonomethylesters und 290 bis 10 Gewichtsteile Dimethylfumarat enthalten, wobei das Gesamtgewicht der Wirkstoffe 20 bis 300 mg beträgt,
ferner als pharmazeutische Zubereitungsform zur oralen Verabreichung in Form von Tabletten oder Kapseln, die dadurch gekennzeichnet sind, daß sie jeweils 10 bis 250 Gewichtsteile des Calciumsalzes des Fumarsäuremonomethylesters, 1 bis 50 Gewichtsteile Dimethylfumarat und 1 bis 50 Gewichtsteile des Zinksalzes des Fumarsäuremonomethylesters enthalten, wobei das Gesamtgewicht der Wirkstoffe 20 bis 300 mg beträgt oder als pharmazeutische Zubereitungsform zur oralen Verabreichung in Form von Tabletten oder Kapseln., die dadurch gekennzeichnet sind, daß sie 10 bis 250 Gewichtsteile des Calciumsalzes des Fumarsäuremonomethylesters, 250 bis 10 Gewichtsteile Dimethylfumarat, 1 bis 50 Gewichtsteile des Magnesiumsalzes des Fumarsäuremonomethylesters und 1 bis 50 Gewichtsteile des Zinksalzes des Fumarsäuremonomethylesters enthalten, wobei das Gesamtgewicht der Wirkstoffe 30 bis 300 mg beträgt, oder auch als
pharmazeutische Zubereitungsform zur oralen Verabreichung, die mit einem magensaftresistenten Überzug versehen sein kann,
als pharmazeutische Zubereitung zur Behandlung der Polyarthritis, Multiplen Sklerose oder von Graft-versus-Host-Reaktionen für die perorale Verabreichung in Form von Pellets, Mikrotabletten, Kapseln, Granulaten und Tabletten, für die kutane und transdermale Verabreichung in Form von Salben, Pflastern oder Lotionen, für die parenterale Verabreichung in Form wäßriger Mikro-Dispersionen, Ö/W-Emulsionen oder öligen Lösungen, für die rektale Verabreichung von Suppositorien oder Mikroklistieren sowie als
pharmazeutische Zubereitung zur Behandlung der oben genannten Autoimmunerkrankungen, die dadurch gekennzeichnet ist, daß sie eine oder mehrere Verbindungen aus der Gruppe der Freien Säuren von Fumarsäuremonoalkylester der allgemeinen Formel 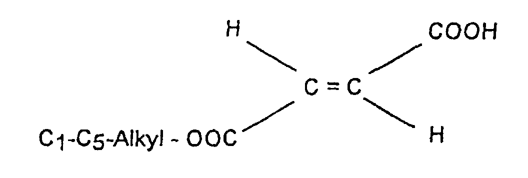 gegebenenfalls im Gemisch mit Dialkylfumarat der Formel 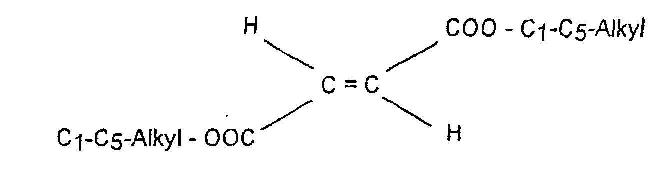
und Trägerstoffe enthält, wobei diese Zubereitungen Fumarsäure in freier Form nicht enthalten oder als
pharmazeutische Zubereitungsform zur oralen Verabreichung in Form von Tabletten, Kapseln oder Mikrotabletten, die dadurch gekennzeichnet sind, daß sie Alkylhydrogenfumarat in einer Menge von 10 bis 300 mg enthalten, wobei das Gesamtgewicht der Wirkstoffe 10 bis 300 mg beträgt oder als
pharmazeutische Zubereitungsform zur oralen Verabreichung in Form von Tabletten, Kapseln oder Mikrotabletten, die dadurch gekennzeichnet sind, daß sie 10 bis 290 Gewichtsteile Alkylhydrogenfumarat und 290 bis 10 Gewichtsteile Dialkylfumarat enthalten, wobei das Gesamtgewicht der Wirkstoffe 20 bis 300 mg beträgt oder als pharmazeutische Zubereitungen, die die freie Säure des Fumarsäuremonomethylesters (Methylhydrogenfumarat) enthalten oder als
pharmazeutische Zubereitungsform zur oralen Verabreichung in Form von Tabletten, Kapseln oder Mikrotabletten, die dadurch gekennzeichnet sind, daß sie jeweils das Methylhydrogenfumarat in einer Menge von 10 bis 300 mg enthalten, wobei das Gesamtgewicht der Wirkstoffe 10 bis 300 mg beträgt oder als
pharmazeutische Zubereitungsform zur oralen Verabreichung in Form von Tabletten, Kapseln oder Mikrotabletten, die 10 bis 290 Gewichtsteile Methylhydrogenfumarat und 290 bis 10 Gewichtsteile Dimethylfumarat enthalten wobei das Gesamtgewicht der Wirkstoffe 20 bis 300 mg beträgt oder schließlich als
pharmazeutische Zubereitung zur Behandlung der oben genannten Autoimmunerkrankungen und insbesondere der Polyarthritis, Multiplen Sklerose oder von Graft-versus-Host-Reaktionen für die perorale Verabreichung in Form von Mikropellets, Mikrotabletten, Kapseln, Granulaten und Tabletten zur kutanen und transdermalen Verabreichung in Form von Salben, Pflastern, Lotionen und Duschmitteln sowie für die parenterale Verabreichung in Form wäßriger Mikro-Dispersionen, Ö/W-Emulsionen oder öligen Lösungen zur rektalen Verabreichung von Suppositorien oder Mikroklistieren.

Nach einer vorzugsweisen Ausgestaltung liegt die Größe bzw der mittlere Durchmesser der Pellets oder Mikrotabletten im Bereich von 300 bis 2000 µm, insbesondere im Bereich von 500µm bis 1500µm oder 1000µm.

Eine weitere besondere Ausgestaltung der Verwendung besteht darin, die Arzneitherapie mit Cyclosporin sequenziell mit einer Applikation der oben bezeichneten Fumarsäurederivate zu alternieren. Das heißt, daß nach ein- bis mehrwöchiger Cyclosporin-Therapie eine ein- bis mehrwöchige Applikation von Fumarsäurederivaten gemäß vorstehender Bedeutung erfolgen kann. Dadurch lassen sich die bekanntlich starken Nebenwirkungen der Langzeitverabreichung von Cyclosporin in unerwarteter Weise drastisch verringern.

Im folgenden werden zur Erläuterung der erfindungsgemäßen Verwendung verschiedene Beispiele für die Herstellung bevorzugter Arzneimittel gegeben:

### Herstellungsbeispiele

### Beispiel 1

### Herstellung von Filmtabletten mit magensaftresistentem Ueberzug enthaltend 100,0 mg Monoethylfumarat-Ca-Salz, entsprechend 71 mg Fumarsäure

10,000 kg Monoethylfumarat-Ca-Salz werden zerkleinert, intensiv gemischt und unter entsprechenden Vorsichtsmassnahmen (Atemmaske, Handschuhe, Schutzanzug etc.) mittels eines Siebes 800 homogenisiert. Anschliessend wird ein Hilfsstoffgemisch folgender Zusammensetzung hergestellt: 21,000 kg Stärkederivat (STA-RX 1500®), 2,000 kg mikrokristalline Cellulose (Avicel PH 101®), 0,600 kg Polyvinylpyrolidon (PVP, Kollidon®25), 4,000 kg Primogel®, 0,300 kg kolloidaler Kieselsäure (Aerosil®).
Das gesamte Pulvergemisch wird mit dem Wirkstoff versetzt, gemischt, mittels eines Siebes 200 homogenisiert und mit einer 2%igen wässrigen Lösung von Polyvinylpyrolidon (PVP, Kollidon®25) auf übliche Weise zu einem Bindemittelgranulat verarbeitet und in trockenem Zustand mit der äusseren Phase gemischt. Diese besteht aus 2,000 kg eines sogenannten FST-Komplexes, enthaltend 80% Talk, 10% Kieselsäure und 10% Magnesiumstearat.
Es wird anschliessend auf übliche Weise zu gewölbten Tabletten von 400 mg Gewicht und 10,0 mm Durchmesser gepresst. Anstelle dieser klassischen Tablettiermethoden können auch andere Methoden zur Herstellung von Tabletten angewendet werden, wie Direkttablettierung sowie feste Dispersionen nach der Schmelzmethode und der Sprühtrocknungsmethode.

### Magensaftresistenz:

Es wird eine Lösung von 2,250 kg Hydroxypropylmethylcellulosephthalat (HPMCP, Pharmacoat HP®50) in einem Lösungsmittelgemisch von 2,50 I demineralisiertem Wasser, 13,00 I Aceton Ph.Helv.VII und 13,00 1 Ethanol 94 Gewichtsprozent gelöst und die Lösung mit 0,240 kg Rizinusöl (Ph.Eur. II) versetzt. Die Lösung wird im Dragierkessel auf traditionelle Weise in Portionen auf die Tablettenkerne aufgeleert oder aufgesprüht bzw. in einem Wirbelschichtapparat entsprechender Konstruktion aufgetragen.
Nach entsprechender Trocknung wird anschliessend der Filmüberzug angebracht. Dieser setzt sich zusammen aus einer Lösung von Eudragit E 12,5%® 4,800 kg, Talcum Ph. Eur. II 0,340 kg, Titan (VI)-oxyd Cronus RN 56® 0,520 kg, Farblack ZLT-2 blau (Siegle) 0,210 kg, und Polyethylenglycol 6000 Ph.Helv.VII 0,120 kg in einem Lösungsmittelgemisch von 8,200 kg 2-Propanol Ph.Helv.VII, 0,060 kg Glycerintriacetat (Triacetin®) und 0,200 kg Aqua demineralisata. Nach homogener Verteilung im Dragierkessel oder Wirbelschichtbett wird getrocknet und auf übliche Weise poliert.

### Beispiel 2

### Herstellung von magensaftresistenten Kapseln, enthaltend 86,5 mg Monoethylfumarat-Ca-Salz und 110,0 mg Dimethylfumarat, entsprechend insgesamt 150 mg Fumarsäure

8,650 kg Monoethylfumarat-Ca-Salz und 11,000 kg Dimethylfumarat werden mit einem Gemisch bestehend aus 15,000 kg Stärke, 6,000 kg Lactose Ph. Helv. VII, 2,000 kg mikrokristalline Cellulose (Avicel®), 1,000 kg Polyvinylpyrolidon (Kollidon®25) und 4,000 kg Primogel® intensiv gemischt und unter entsprechenden Vorsichtsmassnahmen (Atemmaske, Handschuhe, Schutzanzug etc.) mittels eines Siebes 800 homogenisiert.
Das gesamte Pulvergemisch wird mit einer 2%igen wässrigen Lösung von Polyvinylpyrolidon (Kollidon® 25) auf übliche Weise zu einem Bindemittelgranulat verarbeitet und in getrocknetem Zustand mit der äusseren Phase gemischt. Diese besteht aus 0,350 kg kolloidaler Kieselsäure (Aerosil®), 0,500 kg Mg-Stearat und 1,500 kg Talkum Ph. Helv. VII. Das homogene Gemisch wird anschliessend in entsprechende Kapseln in Portionen von 500.0 mg abgefüllt, welche abschliessend auf übliche Weise mit einem magensaftresistenten Ueberzug, bestehend aus Hydroxypropylmethylcellulosestearat und Rizinusöl als Weichmacher versehen werden. Die Abfüllung kann ebenfalls anstelle von Hartgelatinekapseln in entsprechende magensaftresistente Kapseln, bestehend aus einem Gemisch von Celluloseacetatphthalat (CAP) und Hydroxypropylethylcellulosephthalat (HPMCP) erfolgen.

### Beispiel 3

### Herstellung von magensaftresistenten Kapseln, enthaltend 203,0 mg Monoethylfumarat-Ca-Salz, 5,0 mg Monoethyl-furnarat-Mg-Salz und 3,0 mg Monoethylfumarat-Zn-Salz, entsprechend insgesamt 150 mg Fumarsäure

20,300 kg Monoethylfumarat-Ca-Salz sowie 0,500 kg Monoethylfumarat-Mg-Salz und 0,300 kg Monoethylfumarat-Zn-Salz werden zerkleinert, intensiv gemischt und unter entsprechenden Vorsichtsmassnahmen (Atemmaske, Handschuhe, Schutzanzug etc.) mittels eines Siebes 800 homogenisiert. Diesem Wirkstoffgemisch wird ein homogenes Pulvergemisch folgender Zusammensetzung untergemischt: sprühgetrocknete Lactose 12,900 kg, kolloidale Kieselsäure 1,000 kg, mikrokristailine Cellulose (Avicel ®) 2,000 kg, Magnesiumstearat (Fh. Heiv. VII) 1,000 kg und Talk (Ph.Helv.VII) 2,000 kg. Das gesamte Pulvergemisch wird nochmals mittels eines Siebs 200 homogenisiert und anschliessend in Hartgelatinekapseln zu 400 mg Nettogewicht eingefüllt und verschlossen. Das Ueberziehen mit einem magensaftresistentern Ueberzug erfolgt wie in Beispiel 2.

### Beispiel 4

### Herstellung von magensaftresistenten Mikrotabletten in Kapseln, enthaltend 87,0 mg Monoethylfumarat Ca-Salz, 120,0 mg Dimethylfumarat, 5,0 mg Monoethylfumarat Mg-Salz und 3,0 mg Monoethylfumarat Zn-Salz, entsprechend insgesamt 164 mg Fumarsäure ("Forte"-Tabletten)

8,700 kg Monoethlfumarat Ca-Salz, 12.000 kg Dimethylfumarat, 0,500 kg Monoethylfumarat Mg-Salz, 0.30 kg Monoethylfumarat Zn-Salz werden zerkleinert, intensiv gemischt und mittels eines Siebs 800 unter entsprechenden Vorsichtsmassnahmen (Atemmaske, Handschuhe, Schutzanzug, etc.) homogenisiert. Es wird ein Hilfsstoffgemisch folgender Zusammensetzung hergestellt: 18,00 kg Stärkederivat (STA-RX 1500), 0,30 kg Cellulose mikrokristallin (Avicel PH 101), 0,75 kg PVP (Kollidon 120), 4,00 kg Primogel, 0,25 kg Kieselsäure kolloidal (Aerosil). Das gesamte Pulvergemisch wird mit dem Wirkstoffgemisch versetzt und mittels eines Siebs 200 homogenisiert und mit einer 2%igen wässrigen Lösung von Polyvinylpyrrolidon (Kollidon K25) auf übliche Weise zu einem Bindemittelgranulat verarbeitet und in trockenem Zustand mit der äusseren Phase gemischt. diese besteht aus 0,50 kg Mg-Stearat und 1,50 kg Talkum. Das Pulvergemisch wird anschliessend auf üblicher Weise zu gewölbten Mikrotabletten von 10,0 mg Bruttomasse und 2,0 mm Durchmesser gepresst. Anstelle dieser klassischen Tablettiermethode können auch andere Methoden zur Herstellung von Tabletten verwendet werden, wie Direkttablettierung sowie feste Dispersionen nach der Schmelzmethode und die Sprühtrocknungsmethode.
Der magensaftresistente Ueberzug kann in einem klassischen Dragierkessel aufgeleert oder aufgesprüht sowie in einer Wirbelschichtapparatur erfolgen. Zum Erreichen der Magensaftresistenz wird portionsweise eine Lösung von 2,250 kg Hydroxypropylmethylcellulosephthalat (HPMCCP, Pharmacoat HP 50), in einem Gemisch folgender Lösungsmittel aufgelöst: Aceton 13,00 I, Ethanol 94 Gewichtsprozent denaturiert mit 2% Keton 13,50 I und Aqua demineralisata 2,50 I. Zu der fertigen Lösung wird als Weichmacher Rizinusöl 0,240 kg zugegeben und auf übliche Weise in Portionen auf die Tablettenkerne aufgetragen.
Filmcoat: Nach beendeter Trocknung wird anschliessend in der gleichen Apparatur eine Suspension folgender Zusammensetzung als Filmcoat aufgetragen: Talk 0,340 kg, Titan (VI)-oxyd Cronus RN 56 0,400 kg, Farblack L-Rotlack 86837 0,324 kg, Eudragit E 12,5% 4,800 kg und Polyethylenglycol 6000 pH 11 Xl 0,120 kg in einem Lösungsgemisch folgender Zusammensetzung: 2-Propanol 8,170 kg, Aqua demineralisata 0,200 kg und Glycerintriacetat (Triacetin) 0,600 kg.
Die magensaftresistenten Mikrotabletten werden anschliessend in Hartgelatine-Steckkapseln zu 500,0 mg netto Gewicht eingefüllt und verschlossen.

### Beispiel 5

### Herstellung von Filmtabletten mit magensaftresistentem Ueberzug enthaltend 67,0 mg Monoethylfumarat-Ca-Salz, 30,0 mg Dimethylfumarat, 5,0 mg Monoethylfumarat-Mg-Salz und 3,0 mg Monoethylfumarat-Zn-Salz, entsprechend 75 mg Fumarsäure ("Mite"-Tabletten)

3,000 kg Dimethylfumarat, 6,700 kg Monoethylfumarat-Ca-Salz, 0,500 kg Monoethylfumarat-Mg-Salz und 0,300 kg Monoethylfumarat-Zn-Salz werden zerkleinert, intensiv gemischt und unter entsprechenden Vorsichtsmassnahmen (Atemmaske, Handschuhe, Schutzanzug etc.) mittels eines Siebes 800 homogenisiert. Es wird ein Hilfsstoffgemisch folgender Zusammensetzung, auf ähnliche Weise wie unter Beispiel 4 aufgeführt hergestellt, nämlich 30,000 kg Stärkederivat (STA-RX 1500®), 3,000 kg mikrokristalline Cellulose (Avicel PH 101®), 0,750 kg Polyvinylpyrolidon (PVP, Kollidon®25), 4,000 kg Primogel, 0,250 kg kolloidaler Kieselsäure (Aerosil®). Hilfsstoffe und Wirkstoffgemisch werden intensiv gemischt und mittels eines Siebes 200 homogenisiert. Das Ganze wird mit einer 2%igen wässrigen Lösung von Polyvinylpyrolidon (PVP, Kollidon®25) auf übliche Weise zu einem Bindemittelgranulat verarbeitet. Dem getrockneten Granulat wird eine Pulvermischung aus folgenden Hilfsstoffen als äussere Phase zugesetzt: 0,500 kg Mg-Stearat (Ph.Eur.) und 0,800 kg Talk (Ph. Eur. II).
Die homogene Granulatmischung wird zu gewölbten Tablettenkernen von 500,0 mg Gewicht und 11,5 mm Durchmesser auf übliche Weise komprimiert. Neben den Bindemittelmethoden können ebenfalls andere Tablettiermethoden, gemäss den Beispielen 1 und 4, Verwendung finden. Das Ueberziehen der Tablettenkerne mit einem magensaftresistenten Ueberzug sowie mit einem Filmcoat erfolgt sinngemäss wie unter den Beispielen 1 und 4 beschrieben.
Vorzugsweise werden die erfindungsgemässen Zubereitungen peroral in Form von Tabletten oder Kapseln verabreicht, wobei diese festen Einzeldosis-Arzneiformen vorzugsweise mit einem magensaftresistenten Ueberzug versehen sind, der sich nach der Magenpassage im Dünndarmsaft im Dünndarm innerhalb weniger Minuten löst und das aktive Prinzip aus der Arzneiform freisetzt. Zum systemischen Einstieg bzw. Ausstieg ist eine niedrige Dosierung (mite) erforderlich, für die therapeutische Dosierung nach der Einstiegsphase eine höhere Dosierung (forte).

Gegenstand der Erfindung sind neben oral verabreichbaren Präparaten in Form von Kapseln, Granulaten und Tabletten, Präparate für die kutane und transdermale Verabreichung in Form von Salben, Pflastern, Lotionen und Duschmitteln, Präparate für die parenterale Verabreichung in Form wässriger Mikrodispersionen, O/W-Emulsionen oder öliger Lösungen, Präparate für die rektale Verabreichung als Suppositorien oder Mikroklistiere sowie Präparate für die medikamentöse Behandlung von Haaren, Finger- und Zehennägeln.

### Beispiel 6

### Herstellung von Filmtabletten mit magensaftresistentem Ueberzug enthaltend 100,0 mg Monomethylfumarat-Ca-Salz, entsprechend 78 mg Fumarsäure

10,000 kg Monomethylfumarat-Calciumsalz werden zerkleinert, gemischt und mittels eines Siebes 800 unter entsprechenden Vorsichtsmassnahmen (Atemmaske, Handschuhe, Schutzanzug etc.) homogenisiert. Anschliessend wird ein Hilfsstoffgemisch folgender Zusammensetzung hergestellt: 21,000 kg Stärkederivat (STA-RX 1500®), 2,000 kg mikrokristalline Cellulose (Avicel PH 101®), 0,600 kg Polyvinylpyrolidon (PVP, Kollidon®25), 4,000 kg Primogel, 0,300 kg kolloidaler Kieselsäure (Aerosil®). Das gesamte Pulvergemisch wird mit dem Wirkstoff versetzt, gemischt und mittels eines Siebes 200 homogenisiert und mit einer 2%igen wässrigen Lösung von Polyvidonpyrolidon (Kollidon ®K30) auf übliche Weise zu einem Bindemittelgranulat verarbeitet und in trockenem Zustand mit der äusseren Phase gemischt. Diese besteht aus 2,000 kg eines sogenannten FST-Komplexes, enthaltend 80% Talk, 10% Kieselsäure und 10% Magnesiumstearat. Es wird anschliessend auf übliche Weise zu gewölbten Tabletten von 400 mg Gewicht und 10 mm Durchmesser gepresst. Anstelle dieser klassischen Tablettiermethoden können auch andere Methoden zur Herstellung von Tabletten angewendet werden, wie Direkttablettierung sowie feste Dispersionen nach der Schmelzmethode und der Sprühtrocknungsmethode. Das Ueberziehen der Tablettenkerne mit einem magensaftresistenten Ueberzug sowie mit einem Filmcoat erfolgt sinngemäss wie unter den Beispielen 1 und 4 beschrieben.

### Beispiel 7

### Herstellung von Filmtabletten mit magensaftresistentem Ueberzug enthaltend 50,0 mg Monomethylfumarat-Ca-Salz, 50,0 mg Dimethylfumarat, 5,0 mg Monomethylfumarat-Mg-Salz und 3,0 mg Monomethylfumarat-Zn-Salz, entsprechend 85 mg Fumarsäure

5,000 kg Dimethylfumarat, 5,000 kg Monomethylfumarat-Ca-Salz, 0,500 kg Monomethylfumarat-Mg-Salz und 0,300 kg Monomethylfumarat-Zn-Salz werden zerkleinert, intensiv gemischt und unter entsprechenden Vorsichtsmassnahmen (Atemmaske, Handschuhe, Schutzanzug etc.) mittels eines Siebes 800 homogenisiert. Es wird ein Hilfsstoffgemisch folgender Zusammensetzung, auf ähnliche Weise wie unter Beispiel 4 aufgeführt hergestellt, nämlich 19,000 kg Stärkederivat (STA-RX 1500®), 3,000 kg mikrokristalline Cellulose (Avicel PH 101®), 0,750 kg Polyvinylpyrolidon (PVP, Kollidon®120), 4,000 kg Primogel, 0,250 kg kolloidaler Kieselsäure (Aerosil®).
Hilfsstoffe und Wirkstoffgemisch werden intensiv gemischt und mittels eines Siebes 200 homogenisiert. Das Ganze wird mit einer 2%igen wässrigen Lösung von Polyvinylpyrolidon (PVP, Kollidon®25) auf übliche Weise zu einem Bindernittelgranulat verarbeitet und in getrocknetem Zustand mit der äusseren Phase gemischt. Diese besteht aus 0,500 kg Mg-Stearat (Ph. eur.) und 1,500 kg Talk (Ph. Eur. II).
Das ganze Granulat wird anschliessend auf übliche Weise zu gewölbten Tabletten von 400 mg Gewicht und 10 mm Durchmesser gepresst. Anstelle dieser klassischen Tablettiermethoden können auch andere Methoden zur Herstellung von Tabletten angewendet werden, wie Direkttablettierung sowie feste Dispersionen nach der Schmelzmethode und der Sprühtrocknungsmethode.
Das Ueberziehen der Tablettenkerne mit einem magensaftresistenten Ueberzug sowie mit einem Filmcoat erfolgt sinngemäss wie unter den Beispielen 1 und 4 beschrieben.

### Beispiel 8

### Herstellung von Filmtabletten mit magensaftresistentern Ueberzug enthaltend 50,0 mg Mono-n-propylfumarat-Ca-Salz, entsprechend 32,8 mg Fumarsäure

5,000 kg Monopropylfumarat-Calciumsalz werden zerkleinert, gemischt und mittels eines Siebes 800 unter entsprechenden Vorsichtsmassnahmen (Atemmaske, Handschuhe, Schutzanzug etc.) homogenisiert. Anschliessend wird ein Hilfsstoffgemisch folgender Zusammensetzung hergestellt: 25,000 kg Stärkederivat (STA-RX 1500®), 3,000 kg mikrokristalline Cellulose (Avicel PH 101®), 0,600 kg Polyvinylpyrolidone (PVP, Kollidon®25), 4,000 kg Primogel, 0,300 kg kolloidaler Kieselsäure (Aerosil®). Das gesamte Pulvergemisch wird mit dem Wirkstoff versetzt, gemischt, mittels eines Siebes 200 homogenisiert und mit einer 2%igen wässrigen Lösung von Polyvidonpyrolidon (Kollidon ®K30) auf übliche Weise zu einem Bindemittelgranulat verarbeitet und in trockenem Zustand mit der äusseren Phase gemischt. Diese besteht aus 2,000 kg eines sogenannten FST-Komplexes, enthaltend 80% Talk, 10% Kieselsäure und 10% Magnesiumstearat. Es wird anschliessend auf übliche Weise zu gewölbten Tabletten von 400 mg Gewicht und 10 mm Durchmesser gepresst. Anstelle dieser klassischen Tablettiermethoden können auch andere Methoden zur Herstellung von Tabletten angewendet werden, wie Direkttablettierung sowie feste Dispersionen nach der Schmelzmethode und der Sprühtrocknungsmethode. Das Ueberziehen der Tablettenkerne mit einem magensaftresistenten Ueberzug sowie mit einem Filmcoat erfolgt sinngemäss wie unter den Beispielen 1 und 4 beschrieben.

### Beispiel 9

### Herstellung von magensaftresistenten Pellets in Kapseln enthaltend 50,0 mg Monomethylfumarat-Ca-Salz, 5,0 mg Monomethylfumarat-Mg-Salz und 3,0 mg Monomethylfumarat-Zn-Salz, entsprechend 45 mg Fumarsäure

5,000 kg Monomethylfumarat-Ca-Salz, 0,500 kg Monomethylfumarat-Mg-Salz und 0,300 kg Monomethylfumarat-Zn-Salz werden zerkleinert, intensiv gemischt und unter entsprechenden Vorsichtsmassnahmen (Atemmaske, Handschuhe, Schutzanzug etc.) mittels eines Siebes 400 homogenisiert. Daneben wird 2 I einer 20% (m/V) Polyvinylpyrrolidon (Kollidon K30) Lösung in Ethanol vorbereitet. 7,250 kg Nonpareilles Pellets werden in einem Dragierkessel belegt und mit einem Teil der Kollidon K-30 Lösung besprüht bis diese leicht feucht werden. Das Wirkstoffgemisch wird danach portionsweise zugegeben bis zum Auftrocknen der Pellets. Diese Vorgehensweise der Befeuchtung/Auftrocknung wird bis zur endgültigen Zugabe des Wirkstoffgemisches weitergeführt. Der Rest der PVP Lösung wird mit 0,720 kg Eudragit E 12,5% Lösung gemischt und ganz auf die Pellets gesprüht. Die Pellets werden letztlich bis zum vollständigen Austrocknen bewegt. Anstelle dieser Methode können auch andere weitere Methoden zur Pelletherstellung angewendet werden, wie Wirbelschichtcoating, Extrusion/Spherozination Methode. Weiter können auch die Pellets mit den einzelnen Wirkstoffen hergestellt werden und nach Befilmung (siehe unten) in den entsprechenden Verhältnissen zugemischt werden.
Die Pellets werden mit Eudragit S 12,5% Lösung besprüht und mit Talk aufgetrocknet. Nach jedem Besprühungs-/Auftrocknungszyklus wird die Freisetzung des Wirkstoffs gemessen und Eudragit S 12,5% Lösung/Talkum weiter zugegeben bis eine Freisetzung gemäss Spezifikation erhalten wird.
Die magensaftresistenten Pellets werden danach in Kapseln abgefüllt (146 mg Pellets/Kapsel).

### Beispiel 10

### Herstellung von magensaftresistenten Kapseln, enthaltend 50,0 mg Mono-iso-propylfumarat-Ca-Salz, 50,0 mg Di-iso-propylfumarat, 5,0 mg Mono-iso-propylfumarat-Mg-Salz und 3,0 mg Mono-iso-propylfumarat-Zn-Salz, entsprechend 67 mg Fumarsäure

5,000 kg Mono-iso-propylfumarat-Ca-Salz, 5,000 kg Di-iso-propylfumarat, 0,500 kg Mono-iso-propylfumarat-Mg-Salz und 0,300 kg Mono-iso-propylfumarat-Zn-Salz werden zerkleinert, intensiv gemischt und unter entsprechenden Vorsichtsmassnahmen (Atemmaske, Handschuhe, Schutzanzug etc.) mittels eines Siebes 800 homogenisiert. Diesem Wirkstoffgemisch wird ein homogenes Pulvergemisch folgender Zusammensetzung untergemischt: 32,200 kg sprühgetrocknete Lactose, 2,000 kg Cellulose, mikrokristallin (Avicel) und 1,000 kg kolloidale Kieselsäure (Aerosil), 1,000 kg Magnesiumstearat und 2,000 kg Talk. Das gesamte Pulvergemisch wird nochmals mittels eines Siebs 200 homogenisiert und anschliessend in Hartgelatine-Steckkapseln zu 500 mg Nettogewicht eingefüllt und verschlossen.
Diese Kapseln können danach üblicherweise mit einem magensaftresistenten Ueberzug bestehend aus Hydroxypropylmethylcellulosephthalat (HPMCP) und Rizinusöl als Weichmacher, versehen werden. Die Abfüllung kann ebenfalls anstelle von Hartgelatinekapseln in entsprechende magensaftresistente Kapseln, bestehend aus einem Gemisch von Celluloseacetatphthalat (CAP) und Hydroxypropylethylcelluloseacetatphthalat (HPMCP) erfolgen.

### Beispiel 11

### Herstellung von Mikropellets in Kapseln enthaltend 50,0 mg Methylhydrogenfumarat, entsprechend insgesamt 44,6 mg Fumarsäure

5,000 kg Methylhydrogenfumarat werden zerkleinert und mittels eines Siebes 400 unter entsprechenden Vorsichtsmassnahmen (Atemmaske, Handschuhe, Schutzanzug etc.) homogenisiert. Daneben wird 2 1 einer 20% (m/V) Polyvinylpyrrolidon (Kollidon K30) Lösung in Ethanol vorbereitet. 7,250 kg Nonpareilles Pellets werden in einem Dragierkessel belegt und mit einem Teil der Kollidon K-30 Lösung besprüht bis diese leicht feucht werden. Das Wirkstoffgemisch wird danach portionsweise zugegeben bis zum Auftrocknen der Pellets. Diese Vorgehensweise der Befeuchtung/Auftrocknung wird bis zur endgültigen Zugabe des Wirkstoffgemisches weitergeführt. Die Pellets werden letztlich bis zum vollständigen Austrocknen bewegt. Anstelle dieser Methode können auch andere weitere Methoden zur Pelletherstellung angewendet werden, wie Wirbelschichtcoating, Extrusion/Spherozination Methode. Weiter können auch die Pellets mit den einzelnen Wirkstoffen hergestellt werden und nach Befilmung in den entsprechenden Verhältnissen zugemischt werden.
Die Pellets werden danach in Kapseln abgefüllt (126,5 mg Pellets/Kapsel).

Im folgenden wird anhand der Inhibierung der Hämagglutininbildung im Tierversuch die Wirksamkeit der anmeldungsgemäßen Verwendung aufgezeigt und mit einem anerkannten Arzneimittel des Standes der Technik verglichen:

### Untersuchung des Einflusses einer Formulierung gemäß Beispiel 4 und Methylhydrogenfumarat-Ca-Salz nach p.o. Gabe auf die Haemagglutininbildung in Mäusen

Durch die Hemmung der Haemagglutininbildung in Mäusen können immunsuppressive Wirkungen von Substanzen nachgewiesen werden. Der Test beruht auf einer direkten Haemagglutination, bei der es durch spezifische gegen die Oberflächenantigene von Erythrozyten gerichtete Antikörper zu einer sichtbaren Verklumpung von Erythrozyten kommt.

Mäuse werden mit Schafserythrozyten immunisiert (Tag 0), dann erfolgt eine 5malige Applikation der zu testenden Substanz (Tag 0-4) und am 9. Tag nach der Immunisierung werden die Haemagglutininspiegel bestimmt. Eine Reduktion in der Haemagglutininbildung zeigt einen immunsuppressiven Effekt an.

Ziel dieser Untersuchungen war es, den Effekt von einer Formulierung gemäss Beispiel 4 und Methylhydrogenfumarat Ca-Salz - nach p.o. Gabe von je 150, 300 und 600 mg/kg - auf die Haemagglutininbildung in Mäusen zu testen.

In diesem Versuch konnte eine dosisabhängige suppressive Wirkung der Formulierung auf Basis der Mengenverhältnisse der Wirkstoffe gemäß Beispiel 4 auf die Haemagglutininbildung in Mäusen nachgewiesen werden. Der Effekt einer Gesamtdosis von 300 mg/kg dieser Formulierung (Applikation der Wirkstoffkombination in 0,8%iger Suspension in wäßrigem HPMC von gelartiger Konsistenz) gemäß lag noch im normalen Streubereich, dagegen konnte nach Gabe von 600 mg/kg der vorstehend bezeichneten Formulierung reproduzierbar eine Inhibition der Haemagglutininbildung um 29 % nachgewiesen werden.

Auch für Methylhydrogenfumarat Ca-Salz konnte eine dosisabhängige suppressive Wirkung auf die Haemagglutininbildung in Mäusen nachgewiesen werden. Eine Dosis von 300 mg/kg Methylhydrogenfumarat Ca-Salz führte zu einer geringfügigen Reduktion der Haemagglutininbildung, während nach Gabe von 600 mg/kg Methylhydrogenfumarat Ca-Salz reproduzierbar eine Inhibition der Haemagglutininbildung um 38% nachgewiesen werden konnte.

Zum Vergleich wurde ein entsprechendes Experiment mit einem Dosisbereich von 150, 200 und 300 mg/kg Cyclosporin A durchgeführt (Wahl des Dosisbereichs nach Borel et al., 1976¹). Für Cyclosporin konnte bei einer Dosis von 150 mg/kg eine Reduktion der Haemagglutininbildung um 37% nachgewiesen werden. Bei der höchsten Dosis von 300 mg Cyclosporin pro kg wurde eine Hemmung der Haemagglutininbildung von 59% erreicht.
¹J. F. Borel et al., Biological Effects of Cyclosporin A: A New Antilymphocytic Agent, Biological and Medical Research Division Sandoz Ltd, CH-4002 Basle, Switzerland; Agents and Actions, 6/4, 468-475(1976)

Die Ergebnisse dieser Untersuchungen lassen den Schluss zu, dass sowohl eine Formulierung gemäss Beispiel 4 als auch Methylhydrogenfumarat Ca-Salz einen deutlichen immunsuppressiven Effekt ausüben.

Der immunsuppressive Effekt von Cyclosporin ist unter anderem bedingt durch eine Hemmung der Bildung von Th-1-Zellen. Wie in-vitro-Versuche gezeigt haben, bewirken Fumarate eine Verschiebung des Zytokinmusters - vom Typ Th1 zum Typ Th2 Zytokinmuster.

Betrachtet man sowohl die Ergebnisse der In-vivo- als auch der in-Vitro-Versuche, so ergibt sich daraus ein sinnvoller und vor allem unerwartet verbesserter Einsatz von Fumaraten in der Transplantationsmedizin vor allem im Hinblick auf die Langzeiterhaltungstherapie.

| **Untersuchung des Einflusses von einer Formulierung gemäß Beispiel und Methylhydrogenfumarat Ca-Salz nach p.o. Gabe auf die Haemagglutininbildung in Mäusen** Reduktion der Bildung von Serumhaemagglutinin in Mäusen | | | |
|---|---|---|---|
| mg/kg Körpergewicht p.o. | Verhältnis der Serumtiter Kontroll-/Verumgruppe | Suppressionsindex | Hemmung der Haemagglutininbildung in % |
| **Wirkstoffkombination wie in Beispiel 4** | | | |
| 150 | 10,7/12,8 | 0,84 | 16 |
| 300 | 10,8/12,8 | 0,84 | 16 |
| 600 | 9,1/12,8 | 0,71 | 29 |

| **Methylhydrogenfumarat Ca-Salz** | | | |
|---|---|---|---|
| 150 | 11,1/12,8 | 0,87 | 13 |
| 300 | 10,2/12,8 | 0,80 | 20 |
| 600 | 7,9/12,8 | 0,62 | 38 |

| **Cyclosporin A** | | | |
|---|---|---|---|
| 150 | 8,0/12,8 | 0,63 | 37 |
| 200 | 7,1/12,8 | 0,55 | 45 |
| 300 | 5,3/12,8 | 0,41 | 59 |
| p.o. = peroral | | | |

Haemagglutinine: Bezeichnung für Substanzen, die eine Haemagglutination herbeirufen, v.a. agglutinierende Antikörper, Phythaemagglutinine, i. R. von Virusinfektionen (z.B. bei Masern, Mumps Röteln, Influenza, Arbovirosen) gebildete H. und Oberflächenantigene bestimmter Virusarten.

Haemagglutination: durch Haemagglutinine verursachte sichtbare Verklumpung von Erythrozyten; als direkte (aktive) H. durch spez. gegen Oberflächenantigene der Erythrozyten gerichtete Antikörper oder als indirekte (passive) H. nach Beladung von Erythrozyten mit einem Antigen (z.B. Vi-Antigen bei der Typhus-Vi-Haemagglutination, Globulin im Antiglobulintest) durch spez. gegen das entsprechende Antigen gerichtete Antikörper. Die Stärke einer H. wird (z.B. bei serologischen Titration eines hämagglutinierenden Antiserums) mit einem Zahlenwert (Verdünnungsstufe des getesteten Serums, bei der gerade noch eine H. ablesbar ist) angegeben.

Die Arzneitherapie im Zusammenhang mit den erfindungsgemäßen Indikationen ist im Vergleich zur Behandlung mit Stoffen des Standes der Technik, beispielsweise Cyclosporin, das zu massiven Nierenstörungen oder Erkrankungen des lymphoproliferativen Systems führen kann, wogegen Fumarsäurederivate zu lediglich vorübergehenden Störungen und nur selten zu ernstlicheren Nebenwirkungen führen, siehe DMW, 121, (1996), S. 1605-1607. Dieser unerwartete Effekt der erfindungsgemäßen Verwendung ist insbesondere in Anbetracht der stets notwendigen Langzeittherapie und -prophylaxe der Graft-versus-Host-Reaktionen oder Multiplen Sklerose von höchstem Interesse. Bei der Kombination von Cyclosporin mit den Fumarsäurederivaten lassen sich die toxischen Nebenwirkungen der erstgenannten Verbindungen in unerwarteter Weise erheblich reduzieren. Die erfindungsgemäße Verwendung ist darüber hinaus auch bei der Substitution der bekanntermaßen mit starken Nebenwirkungen verbundenen Kortikoidtherapie von größter Bedeutung.

## Patentansprüche

1. Verwendung einer oder mehrerer Verbindungen aus der Gruppe der Natrium-, Kalium-, Calcium-, Magnesium-, Zink- und Eisensalze von Fumarsäuremonoalkylestern der allgemeinen Formel 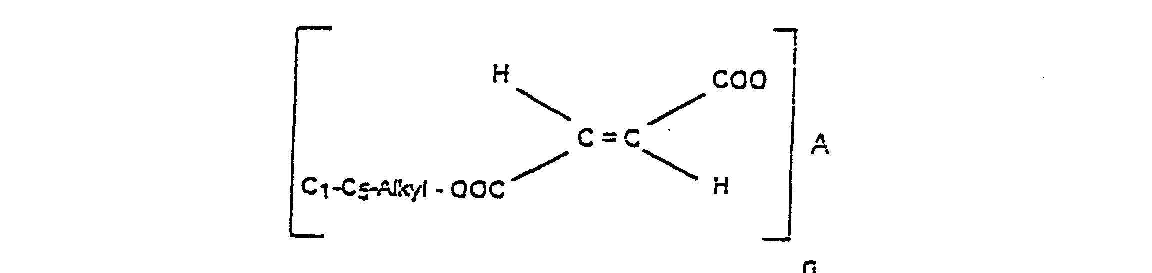 gegebenenfalls im Gemisch mit Dialkylfumarat der Formel 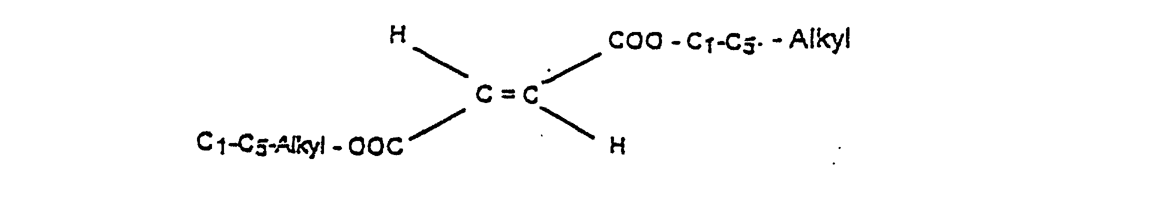 wobei A ein zweiwertiges Kation der Reihe Ca, Mg, Zn oder Fe bzw. ein einwertiges Kation aus der Reihe Kalium oder Natrium und n die Zahl 1 oder 2 je nach Art des Kations bedeuten,
oder
einer oder mehrerer Verbindungen der Reihe der Alkylhydrogenfumarate der allgemeinen Formel 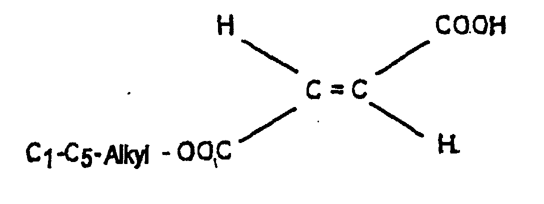 gegebenenfalls im Gemisch mit Dialkylfumarat der Formel 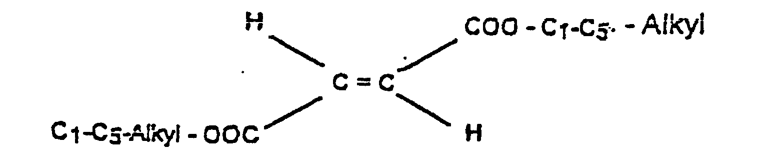 und gegebenenfalls üblichen pharmazeutischen Hilfs- und Trägerstoffen zur Herstellung eines Arzneimittels für die Behandlung einer Autoimmunerkrankung, ausgewählt aus der Gruppe bestehend aus der Polyarthritis, Multiplen Sklerose, Graft-versus-Host-Reaktionen, des juvenilen Diabetes, der Hashimoto-Tyreoiditis, der Grave's disease (Graves Krankheit oder Basedow Krankheit), des systemischen Lupus erythematodes (SLE), des Sjögren Syndroms (Sjogren's Syndrome), der perniziösen Anämie und der chronischen aktiven (= lupoiden) Hepatitis.

2. Verwendung nach Anspruch 1, **dadurch gekennzeichnet, dass** es sich um das Calciumsalz des Fumarsäuremonomethylesters oder des Fumarsäuremonoethylesters handelt.

3. Verwendung nach Anspruch 1, **dadurch gekennzeichnet, dass** es sich um eines oder mehrere aus dem Calcium-, Magnesium- und Zinksalz des Fumarsäuremonoethylesters im Gemisch mit Dimethylfumarat handelt.

4. Verwendung nach Anspruch 1 zur oralen Verabreichung in Form von Tabletten oder Kapseln, **dadurch gekennzeichnet, dass** es sich um das Calciumsalz des Fumarsäuremonoalkylesters in einer Menge von 10 mg bis 300 mg handelt, wobei das Gesamtgewicht der Wirkstoffe 10 bis 300 mg beträgt.

5. Verwendung nach Anspruch 1 zur oralen Verabreichung in Form von Tabletten oder Kapseln, **dadurch gekennzeichnet, dass** es sich um 10 bis 290 Gewichtsteile des Calciumsalzes des Fumarsäuremonoalkylesters und 290 bis 10 Gewichtsteile Dimethylfumarat handelt, wobei das Gesamtgewicht der Wirkstoffe 20 bis 300 mg beträgt.

6. Verwendung nach Anspruch 1 zur oralen Verabreichung in Form von Tabletten oder Kapseln, **dadurch gekennzeichnet, dass** es sich um 10 bis 250 Gewichtsteile des Calciumsalzes des Fumarsäuremonoalkylesters, 1 bis 50 Gewichtsteile Dimethylfumarat und 1 bis 50 Gewichtsteile des Zinksalzes des Fumarsäuremonoalkylesters handelt, wobei das Gesamtgewicht der Wirkstoffe 20 bis 300 mg beträgt.

7. Verwendung nach Anspruch 1 zur oralen Verabreichung in Form von Tabletten oder Kapseln, **dadurch gekennzeichnet, dass** es sich um 10 bis 250 Gewichtsteile des Calciumsalzes des Fumarsäuremonoalkylesters, 250 bis 10 Gewichtsteile Dimethylfumarat, 1 bis 50 Gewichtsteile des Magnesiumsalzes des Fumarsäuremonoalkylesters und 1 bis 50 Gewichtsteile des Zinksalzes des Fumarsäuremonoalkylesters handelt, wobei das Gesamtgewicht der Wirkstoffe 30 bis 300 mg beträgt.

8. Verwendung nach einem der Ansprüche 1 bis 7, **dadurch gekennzeichnet, dass** das Arzneimittel in Form von Mikrotabletten oder Pellets vorliegt, deren Größe bzw. mittlerer Durchmesser im Bereich von 300 bis 2000 µm, insbesondere im Bereich von 500 µm bis 1500 µm liegt.

9. Verwendung nach Anspruch 8, **dadurch gekennzeichnet, dass** die Mikrotabletten oder Pellets mit einem magensaftresistenten Überzug versehen sind.

10. Verwendung nach Anspruch 1 einer oder mehrerer Verbindungen aus der Gruppe der Natrium-, Kalium-, Calcium-, Magnesium-, Zink- und Eisensalze von Fumarsäuremonoalkylestern der allgemeinen Formel 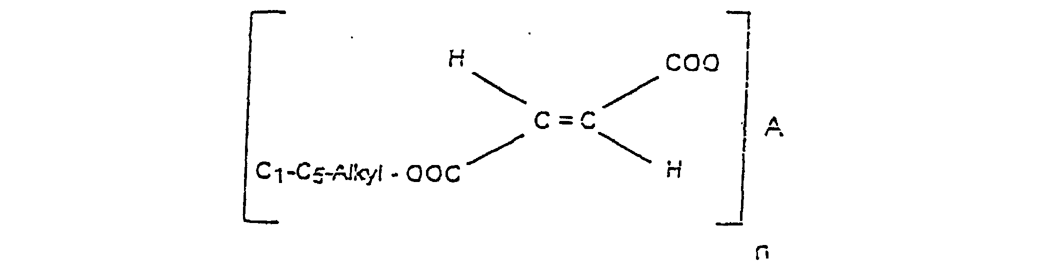 gegebenenfalls im Gemisch mit Dialkylfumarat der Formel 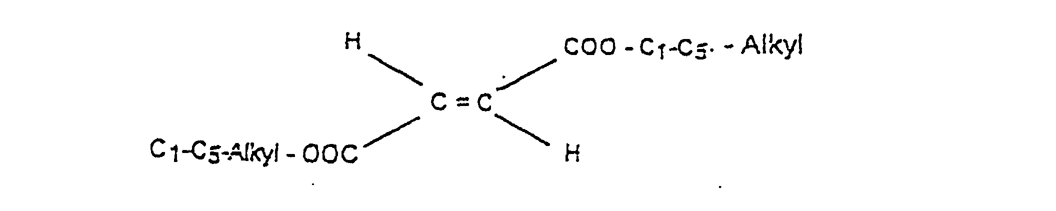 wobei A ein zweiwertiges Kation der Reihe Ca, Mg, Zn oder Fe bzw. ein einwertiges Kation aus der Reihe Kalium oder Natrium und n die Zahl 1 oder 2 je nach Art des Kations bedeuten, oder
einer oder mehrerer Verbindungen der Reihe der Alkylhydrogenfumarate der allgemeinen Formel 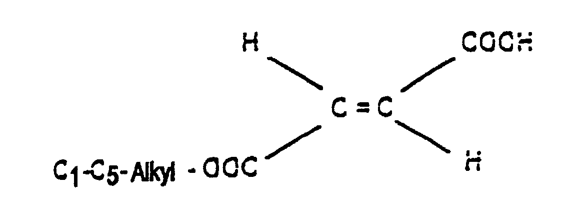 gegebenenfalls im Gemisch mit Dialkylfumarat der Formel 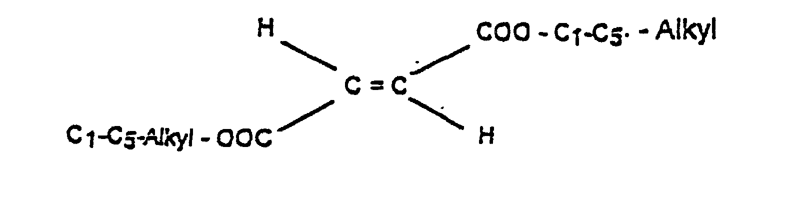 und gegebenenfalls üblicher pharmazeutischer Hilfs- und Trägerstoffe zur Herstellung einer pharmazeutischen Zubereitung in Form von Pellets oder Mikrotabletten zur Behandlung einer Autoimmunerkrankung, ausgewählt aus der Gruppe bestehend aus der Polyarthritis, Multiplen Sklerose, Graft-versus-Host-Reaktionen, dem juvenilen Diabetes, der Hashimoto-Tyreoiditis, der Grave's disease (Graves Krankheit oder Basedow Krankheit), dem systemischen Lupus erythematodes (SLE), dem Sjögren Syndrom (Sjogren's Syndrome), der perniziösen Anämie und der chronischen aktiven (= lupoiden) Hepatitis.

11. Verwendung nach Anspruch 10, **dadurch gekennzeichnet, dass** es sich um das Calciumsalz des Fumarsäuremonomethylesters oder des Fumarsäuremonoethylesters handelt.

12. Verwendung nach Anspruch 10, **dadurch gekennzeichnet, dass** es sich um eines oder mehrere aus dem Calcium-, Magnesium- und Zinksalz des Fumarsäuremonoethylesters im Gemisch mit Dimethylfumarat handelt.

13. Verwendung nach Anspruch 10, wobei es sich um das Calciumsalz des Fumarsäuremonoalkylesters in einer Menge von 10 mg bis 300 mg handelt und wobei das Gesamtgewicht der Wirkstoffe 10 bis 300 mg beträgt.

14. Verwendung nach Anspruch 10, wobei es sich um 10 bis 290 Gewichtsteile des Calciumsalzes des Fumarsäuremonoalkylesters und 290 bis 10 Gewichtsteile Dimethylfumarat handelt und das Gesamtgewicht der Wirkstoffe 20 bis 300 mg beträgt.

15. Verwendung nach Anspruch 10, wobei es sich um 10 bis 250 Gewichtsteile des Calciumsalzes des Fumarsäuremonoalkylesters, 1 bis 50 Gewichtsteile Dimethylfumarat und 1 bis 50 Gewichtsteile des Zinksalzes des Fumarsäuremonoalkylesters handelt und das Gesamtgewicht der Wirkstoffe 20 bis 300 mg beträgt.

16. Verwendung nach Anspruch 10, wobei es sich um 10 bis 250 Gewichtsteile des Calciumsalzes des Fumarsäuremonoalkylesters, 250 bis 10 Gewichtsteile Dimethylfumarat, 1 bis 50 Gewichtsteile des Magnesiumsalzes des Fumarsäuremonoalkylesters und 1 bis 50 Gewichtsteile des Zinksalzes des Fumarsäuremonoalkylesters handelt und das Gesamtgewicht der Wirkstoffe 30 bis 300 mg beträgt.

17. Verwendung nach einem der Ansprüche 10 bis 16, **dadurch gekennzeichnet, dass** die Größe bzw. der mittlere Durchmesser der Pellets oder Mikrotabletten im Bereich von 300 µm bis 2000 µm liegt.

18. Verwendung nach den Ansprüchen 10 bis 17, **dadurch gekennzeichnet, dass** die Dosiseinheiten der Arzneimittel mit einem magensaftresistenten Überzug versehen sind.

## Claims

1. The use of one several compounds selected from the group consisting of sodium, potassium, calcium, magnesium, zinc and iron salts of fumaric acid monoalkyl esters of the general formula 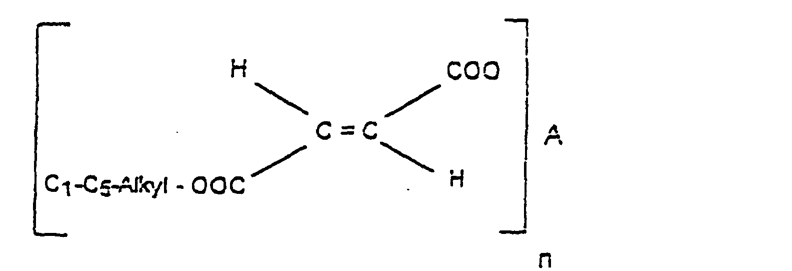 optionally in combination with dialkyl fumarate of the formula 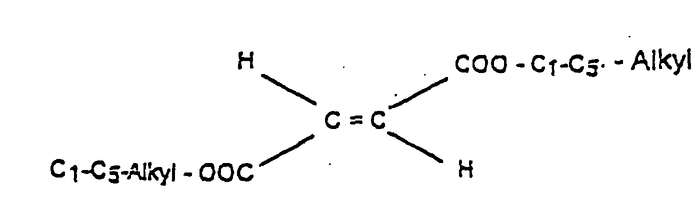 wherein A is a bivalent cation from the series consisting of Ca, Mg, Zn or Fe or a monovalent cation from the series consisting of potassium or sodium respectively, and n denotes the numeral 1 or 2 depending on the type of cation,
or
one or several compounds from the series consisting of alkyl hydrogen fumarates of the general formula 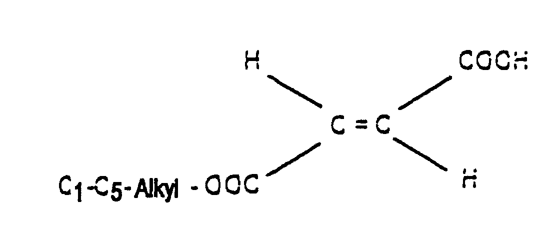 optionally in combination with dialkyl fumarate of the formula 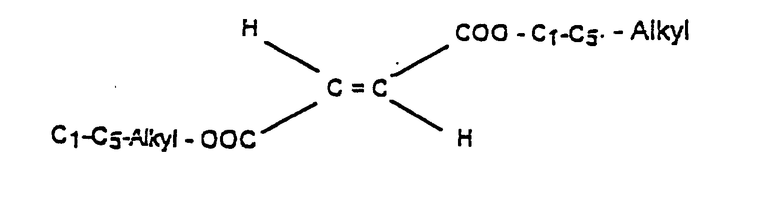 and, optionally, commonly used pharmaceutical excipients and vehicles for manufacture of a pharmaceutical composition for the treatment of an autoimmune disease selected from the group consisting of polyarthritis, multiple sklerosis, graft-versus-host reactions, juvenile diabetes, Hashimoto tyroiditis, Grave's diseases, systemic Lupus erythematodes (SLE), Sjogren's Syndrome, pernicious anaemia and chronic active (lupoid) hepatitis.

2. The use according to claim 1, wherein the calcium salt of the fumaric acid monomethyl ester or fumaric acid monoethyl ester is used.

3. The use according to claim 1, wherein one or several compounds selected from the calcium, magnesium and zinc salt of the fumaric acid monoethyl ester is used in admixture with dimethyl fumarate.

4. The use according to claim 1 for manufacture of a pharmaceutical composition for oral administration in the form of tablets or capsules, wherein the calcium salt of the fumaric acid monoalkyl ester is used in an amount of 10 to 300 mg, the total weight of the active ingredients being 10 to 300 mg.

5. The use according to claim 1 for manufacture of a pharmaceutical composition for oral administration in the form of tablets or capsules, wherein 10 to 290 parts by weight of the calcium salt of the fumaric acid monoalkyl ester and 290 to 10 parts by weight of dimethyl fumarate are used, the total weight of the active ingredients being 20 to 300 mg.

6. The use according to claim 1 for manufacture of a pharmaceutical composition for oral administration in the form of tablets or capsules, wherein 10 to 250 parts by weight of the calcium salt of the fumaric acid monoalkyl ester, 1 to 50 parts by weight of dimethyl fumarate and 1 to 50 parts by weight of the zinc salt of the fumaric acid monoalkyl ester are used, the total weight of the active ingredients being 20 to 300 mg.

7. The use according to claim 1 for preparing a pharmaceutical composition for oral administration in the form of tablets or capsules, wherein 10 to 250 parts by weight of the calcium salt of the fumaric acid monoalkyl ester, 250 to 10 parts by weight of dimethyl fumarate, 1 to 50 parts by weight of the magnesium salt of the fumaric acid monoalkyl ester and 1 to 50 parts by weight of the zinc salt of the fumaric acid monoalkyl ester are used, the total weight of the active ingredients being 30 to 300 mg.

8. The use according to any of the claims 1 to 7, wherein the pharmaceutical composition is in the form of microtablets or pellets, the size or the mean diameter, respectively, of which is in the range of 300 to 2,000 µm, especially in the range of 500 µm to 1,500 µm.

9. The use according to any one of claims 1 to 8, wherein microtablets or pellets are provided with an enteric coating.

10. The use according to claim 1 of one or several compounds selected from the group consisting of sodium, potassium, calcium, magnesium, zinc and iron salts of fumaric acid monoalkyl esters of the general formula 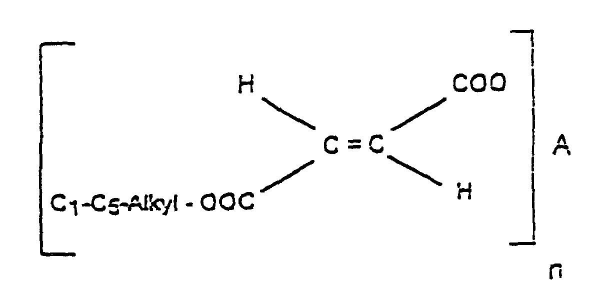 optionally in combination with dialkyl fumarate of the formula 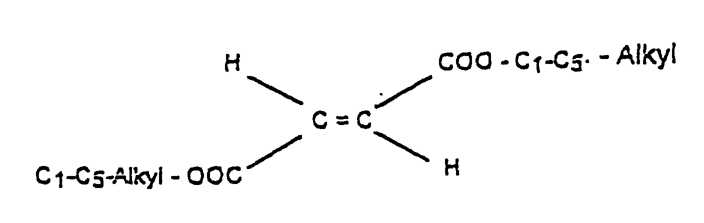 wherein A is a bivalent cation from the series consisting of Ca, Mg, Zn or Fe or a monovalent cation from the series consisting of potassium or sodium respectively, and n denotes the numeral 1 or 2 depending on the type of cation,
or
one or several compounds from the series consisting of alkyl hydrogen fumarates of the general formula 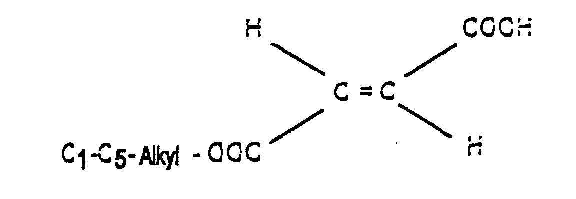 optionally in combination with dialkyl fumarate of the formula 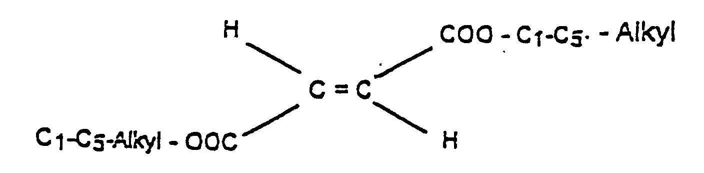 and, optionally, commonly used pharmaceutical excipients and vehicles for manufacture of a pharmaceutical composition in the form of pellets or microtablets for the treatment of an autoimmune disease selected from the group consisting of polyarthritis, multiple sclerosis, graft-versus-host reactions, juvenile diabetes, Hashimoto tyroiditis, Grave's diseases, systemic Lupus erythematodes (SLE), Sjogren's Syndrome, pernicious anaemia and chronic active (lupoid) hepatitis.

11. The use according to claim 10, wherein calcium salt of the fumaric acid monomethyl ester or fumaric acid monoethyl ester is used.

12. The use according to claim 10, wherein one or several compounds selected from the calcium, magnesium and zinc salt of the fumaric acid monoethyl ester is used in admixture with dimethyl fumarate.

13. The use according to claim 10, wherein the calcium salt of the fumaric acid monoalkyl ester is used in an amount of 10 to 300 mg, the total weight of the active ingredients being 10 to 300 mg.

14. The use according to claim 10, wherein 10 to 290 parts by weight of the calcium salt of the fumaric acid monoalkyl ester and 290 to 10 parts by weight of dimethyl fumarate are used, the total weight of the active ingredients being 20 to 300 mg.

15. The use according to claim 10, wherein 10 to 250 parts by weight of the calcium salt of the fumaric acid monoalkyl ester, 1 to 50 parts by weight of dimethyl fumarate and 1 to 50 parts by weight of the zinc salt of the fumaric acid monoalkyl ester are used, the total weight of the active ingredients being 20 to 300 mg.

16. The use according to claim 1, wherein 10 to 250 parts by weight of the calcium salt of the fumaric acid monoalkyl ester, 250 to 10 parts by weight of dimethyl fumarate, 1 to 50 parts by weight of the magnesium salt of the fumaric acid monoalkyl ester and 1 to 50 parts by weight of the zinc salt of the fumaric acid monoalkyl ester are used, the total weight of the active ingredients being 30 to 300 mg.

17. The use according to claim 10 to 16, wherein the size or the mean diameter, respectively, of pellets or microtablets is in the range of 300 µm to 2000 µm.

18. The use according to claim 10 to 17, wherein said dosage units are provided with an enteric coating.

## Revendications

1. Utilisation d'un ou de plusieurs composés du groupe des sels de sodium, potassium, calcium, magnésium, zinc et fer de fumarates de monoalkyle de formule générale 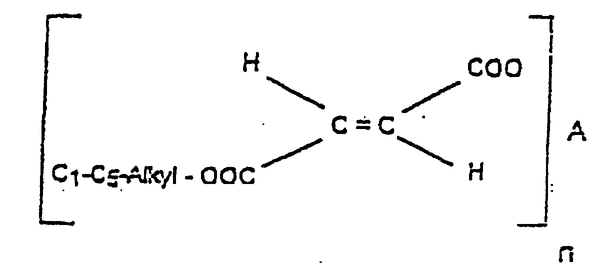 éventuellement en mélange avec du fumarate de dialkyle de formule 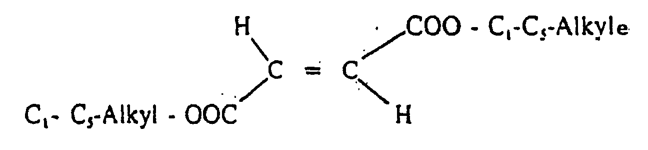 dans laquelle A représente un cation divalent choisi parmi Ca, Mg, Zn ou Fe ou un cation monovalent choisi parmi le potassium ou le sodium et n représente le nombre 1 ou 2 selon le type du cation
ou
d'un ou de plusieurs composés choisis parmi les fumarates de monoalkyle de formule générale 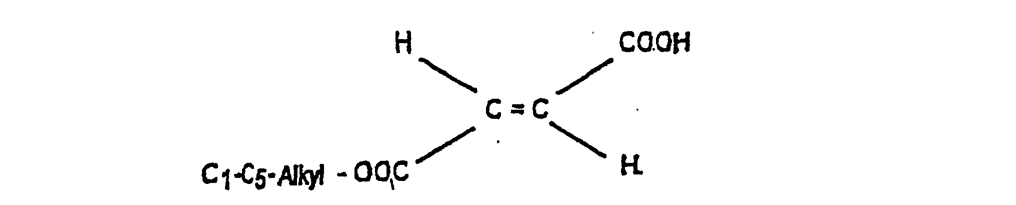 éventuellement en mélange avec du fumarate de dialkyle de formule 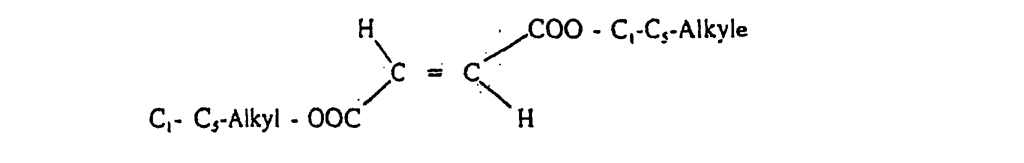 et éventuellement des adjuvants et supports pharmaceutiques usuels pour la fabrication d'un médicament pour le traitement d'une maladie auto-immune, choisie parmi la polyarthrite, les scléroses multiples, les réactions de greffon contre l'hôte, le diabète juvénile, la thyroïdite de Hashimoto, la maladie de Grave (maladie de Grave ou maladie de Basedow), le lupus érythémateux systémique (SLE), le syndrome de Sjögren (Sjögren's Syndrome), l'anémie pernicieuse et l'hépatite chronique active (= lupoïde).

2. Utilisation selon la revendication 1, **caractérisée en ce qu'**il s'agit du sel de calcium de l'hémifumarate de méthyle ou de l'hémifumarate d'éthyle.

3. Utilisation selon la revendication 1, **caractérisée en ce qu'**il s'agit d'un ou de plusieurs composés parmi le sel de calcium, de magnésium et de zinc de l'hémifumarate d'éthyle en mélange avec le fumarate de diméthyle.

4. Utilisation selon la revendication 1 pour l'administration orale sous forme de comprimés ou de capsules, **caractérisée en ce qu'**il s'agit du sel de calcium du fumarate de monoalkyle en une quantité de 10 mg à 300 mg, dans laquelle la masse totale des substances actives est de 10 à 300 mg.

5. Utilisation selon la revendication 1 pour l'administration orale sous forme de comprimés ou de capsules, **caractérisée en ce qu'**il s'agit de 10 à 290 parties en poids du sel de calcium du fumarate de monoalkyle et de 290 à 10 parties en poids de fumarate de diméthyle, dans laquelle la masse totale des substances actives est de 20 à 300 mg.

6. Utilisation selon la revendication 1 pour l'administration orale sous forme de comprimés ou de capsules, **caractérisée en ce qu'**il s'agit de 10 à 250 parties en poids du sel de calcium du fumarate de monoalkyle, de 1 à 50 parties en poids de fumarate de diméthyle et de 1 à 50 parties en poids du sel de zinc du fumarate de monoalkyle, dans laquelle la masse totale des substances actives est de 20 à 300 mg.

7. Utilisation selon la revendication 1 pour l'administration orale sous forme de comprimés ou de capsules, **caractérisée en ce qu'**il s'agit de 10 à 250 parties en poids du sel de calcium du fumarate de monoalkyle, de 250 à 10 parties en poids de fumarate de diméthyle, de 1 à 50 parties en poids du sel de magnésium du fumarate de monoalkyle et de 1 à 50 parties en poids du sel de zinc du fumarate de monoalkyle, dans laquelle la masse totale des substances actives est de 30 à 300 mg.

8. Utilisation selon une des revendications 1 à 7, **caractérisée en ce que** le médicament se présente sous forme de microcomprimés ou de pastilles, dont le diamètre moyen se situe dans la gamme de 300 à 2000 µm, en particulier dans la gamme de 500 µm à 1500 µm.

9. Utilisation selon la revendication 8, **caractérisée en ce que** les microcomprimés ou les pastilles sont munis d'un revêtement résistant au suc gastrique.

10. Utilisation selon la revendication 1 d'un ou de plusieurs composés du groupe des sels de sodium, potassium, calcium, magnésium, zinc et fer des fumarates de monoalkyle de formule générale 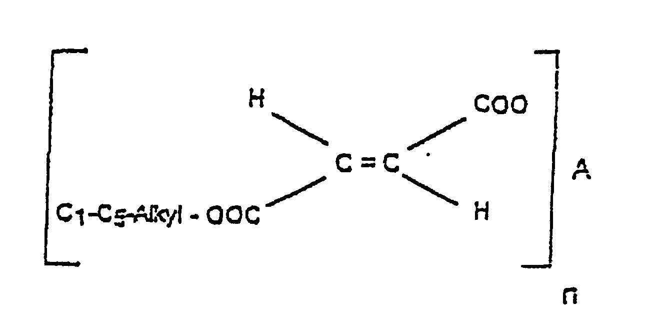 éventuellement en mélange avec du fumarate de dialkyle de formule 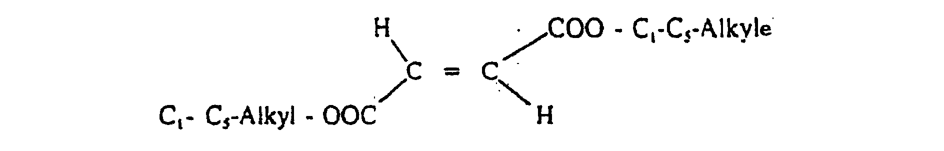 dans laquelle A représente un cation divalent choisi parmi Ca, Mg, Zn ou Fe ou un cation monovalent choisi parmi le potassium ou le sodium et n représente le nombre 1 ou 2 selon le type du cation,
ou
d'un ou de plusieurs composés choisis parmi les fumarates de monoalkyle de formule générale 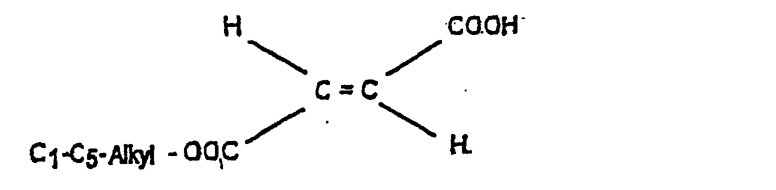 éventuellement en mélange avec du fumarate de dialkyle de formule 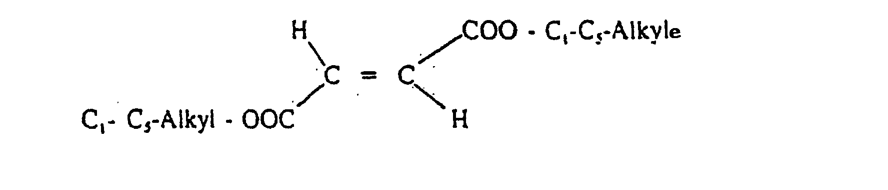 et éventuellement des adjuvants et supports pharmaceutiques usuels pour la fabrication d'une préparation pharmaceutique sous forme de pastilles ou de microcomprimés pour le traitement d'une maladie auto-immune, choisie parmi la polyarthrite, les scléroses multiples, les réactions de greffon contre l'hôte, le diabète juvénile, la thyroïdite de Hashimoto, la maladie de Grave (maladie de Grave ou maladie de Basedow), le lupus érythémateux systémique (SLE), le syndrome de Sjögren (Sjögren's Syndrome), l'anémie pernicieuse et l'hépatite chronique active (= lupoïde).

11. Utilisation selon la revendication 10, **caractérisée en ce qu'**il s'agit du sel de calcium de l'hémifumarate de méthyle ou d'éthyle.

12. Utilisation selon la revendication 10, **caractérisée en ce qu'**il s'agit d'un ou de plusieurs sels de calcium, de magnésium et de zinc de l'hémifumarate d'éthyle en mélange avec le fumarate de diméthyle.

13. Utilisation selon la revendication 10, dans laquelle il s'agit du sel de calcium de l'hémifumarate d'alkyle en une quantité de 10 mg à 300 mg et dans laquelle la masse globale des substances actives est de 10 à 300 mg.

14. Utilisation selon la revendication 10, dans laquelle il s'agit de 10 à 290 parties en poids du sel de calcium du fumarate de monoalkyle et de 290 à 10 parties en poids de fumarate de diméthyle et la masse globale des substances actives est de 20 à 300 mg.

15. Utilisation selon la revendication 10, dans laquelle il s'agit de 10 à 250 parties en poids du sel de calcium du fumarate de monoalkyle, de 1 à 50 parties en poids de fumarate de diméthyle et de 1 à 50 parties en poids du sel de zinc du fumarate de monoalkyle et la masse globale des substances actives est de 20 à 300 mg.

16. Utilisation selon la revendication 10, dans laquelle il s'agit de 10 à 250 parties en poids du sel de calcium du fumarate de monoalkyle, de 250 à 10 parties en poids de fumarate de diméthyle, de 1 à 50 parties en poids du sel de magnésium du fumarate de monoalkyle et de 1 à 50 parties en poids du sel de zinc du fumarate de monoalkyle et la masse globale des substances actives est de 30 à 300 mg.

17. Utilisation selon l'une des revendications 10 à 16, **caractérisée en ce que** la taille ou le diamètre moyen des pastilles ou des microcomprimés se situe dans la gamme de 300 µm à 2000 µm.

18. Utilisation selon les revendications 10 à 17, **caractérisée en ce que** les doses unitaires du médicament sont munies d'un revêtement résistant au suc gastrique.
